# FASCICULE DE BREVET EUROPEEN

(11) **EP 1 515 712 B1**
(45) Date de publication et mention de la délivrance du brevet: **25.12.2019**
(21) Numéro de dépôt: 03760772.8
(22) Date de dépôt: 23.06.2003
(51) Int. Cl.: A61K 31/185, A61K 31/195, A61K 8/46, A61K 8/63, A61Q 7/00, A61K 8/86, A61K 45/06, A61K 9/00, A61K 9/20, A61K 9/48, A23L 33/105, A23L 33/115, A23L 33/175, A61P 17/14

(54) **UTILISATION DE LA TAURINE OU DE DERIVES POUR LE TRAITEMENT DE L'ALOPECIE**
VERWENDUNG VON TAURIN ODER DESSEN DERIVATEN ZUR BEHANDLUNG VON HAARAUSFALL
USE OF TAURINE FOR THE TREATMENT OF ALOPECIA

(30) Priorité: 21.06.2002 FR 0207763; 21.06.2002 FR 0207764; 21.06.2002 FR 0207765
(43) Date de publication de la demande: 23.03.2005
(60) Demande divisionnaire: 10184434.8
(73) Titulaire: L'Oréal, 75008 Paris (FR); Société des Produits Nestlé S.A., 1800 Vevey (CH)
(72) Inventeur: DURANTON, Albert, 78600 MAISONS-LAFFITTE (FR); BRETON, Lionel, 78000 Versailles (FR)
(74) Mandataire: Nony
(86) Numéro de dépôt international: PCT/FR2003/001919
(87) Numéro de publication internationale: WO 2004/000293

(56) Documents cités:
- EP-A- 0 652 012
- WO-A-00/07607
- WO-A-91/11117
- WO-A-94/02166
- WO-A-95/26646
- WO-A-02/096221
- DE-U- 20 204 844
- FR-A- 2 734 477
- US-A- 5 223 285
- US-A- 5 700 782
- US-A- 5 895 652
- US-A- 5 976 568
- US-A- 6 103 756
- US-B1- 6 261 589
- DATABASE CAPLUS [Online] CHEMICAL ABSTRACTS SERVICE, COLUMBUS, OHIO, US; XP002230967 extrait de 2002:240556 accession no. STN Database accession no. 136:267900 cité dans la demande & WO 02/24189 A (SHISEIDO COMPANY) 28 mars 2002 (2002-03-28)
- DATABASE WPI Week 200260 Derwent Publications Ltd., London, GB; AN 2002-560763 XP002230968 & JP 2002 128651 A (KOSE KK)
- PATENT ABSTRACTS OF JAPAN vol. 2000, no. 01, 31 janvier 2000 (2000-01-31) & JP 11 292753 A (DOWA YAKUSHOU KK;SOGO PHARMACEUT CO LTD), 26 octobre 1999 (1999-10-26)
- DATABASE WPI Week 200147 Derwent Publications Ltd., London, GB; AN 2001-438387 XP002230969 & KR 2001 000 741 A (CHOI SEONG KON ;KWON LEE SUN (KR)) 5 janvier 2001 (2001-01-05)
- DATABASE WPI Section Ch, Week 199719 Derwent Publications Ltd., London, GB; Class B02, AN 1997-209224 XP002230970 & JP 09 059154 A (ITOEN KK) 4 mars 1997 (1997-03-04)
- DATABASE WPI Section Ch, Week 199727 Derwent Publications Ltd., London, GB; Class B05, AN 1997-292424 XP002230971 & JP 09 107917 A (SNOW BRAND MILK PROD CO LTD) 28 avril 1997 (1997-04-28)
- DATABASE WPI Section Ch, Week 200220 Derwent Publications Ltd., London, GB; Class B05, AN 2002-148600 XP002230972 & CN 1 322 552 A (UNIV SANYE SCI & TECHNOLOGY CO LTD SHANG) 21 novembre 2001 (2001-11-21)
- DATABASE WPI Section Ch, Week 200301 Derwent Publications Ltd., London, GB; Class B04, AN 2003-010729 XP002260876 & KR 2002 041 348 A (AMINOGEN CO LTD) 1 juin 2002 (2002-06-01)
- DATABASE WPI Section Ch, Week 200138 Derwent Publications Ltd., London, GB; Class B04, AN 2001-365618 XP002260877 & RU 2 165 719 C (AS USSR FAR E PACIFIC OCEAN BIOORG INST) 27 avril 2001 (2001-04-27)
- DATABASE WPI Week 199826 Derwent Publications Ltd., London, GB; AN 1998-289843 XP002260878 & JP 10 099048 A (SNOW BRAND MILK PROD. CO LTD) 21 avril 1998 (1998-04-21)
- DATABASE WPI Week 200339 Derwent Publications Ltd., London, GB; AN 2003-407325 XP002260879 & JP 2002 330748 A (NAKAMURA N.) 19 novembre 2002 (2002-11-19)
- DATABASE WPI Week 199544 Derwent Publications Ltd., London, GB; AN 1995-340175 XP002260880 & JP 07 233046 A (KOSE KK) 5 septembre 1995 (1995-09-05)
- Frucor Beverages Ltd.: "Enquiry V energy drink - answer 1", , 20 July 2015 (2015-07-20), XP055295852, Retrieved from the Internet: URL:http://nul.org [retrieved on 2016-08-17]
- Frucor Beverages Ltd.: "Enquiry V energy drink - answer 2", , 20 July 2015 (2015-07-20), XP055295853, Retrieved from the Internet: URL:http://nul.org [retrieved on 2016-08-17]
- Frucor Beverages Ltd.: "V Green label", , 20 July 2015 (2015-07-20), XP055295851, Retrieved from the Internet: URL:http://nul.org [retrieved on 2016-08-17]

## Description

La présente invention concerne principalement l'utilisation cosmétique de la taurine et/ou de l'hypotaurine et/ou leurs sels acceptables, en association avec au moins un polyphénol et/ou un extrait en comportant, pour une administration par voie orale, pour maintenir une belle chevelure en agissant sur la densité capillaire et en réduisant l'hétérogénéité des diamètres capillaires ; le dit polyphénol étant choisi parmi une procyanidine, une proanthocyanidine, un mélange de monomères catéchines avec des oligomères de procyanidines (OPC), ainsi que leurs mélanges.

La présente invention concerne aussi principalement une composition pour l'absorption orale caractérisée en ce qu'elle comprend au moins un polyphénol choisi parmi une procyanidine, une proanthocyanidine, un mélange de monomères catéchines avec des oligomères de procyanidines (OPC), ainsi que leurs mélanges et/ou un extrait en comportant, en association avec de la taurine et/ou l'hypotaurine et/ou leurs sels acceptables.

La présente invention concerne aussi principalement une association de taurine et/ou d'hypotaurine et/ou de sels acceptables, avec au moins un polyphénol et/ou un extrait en comportant, destinée à une utilisation pour administration orale dans le traitement et la prévention de l'alopécie ; le dit polyphénol étant choisi parmi une procyanidine, une proanthocyanidine, un mélange de monomères catéchines avec des oligomères de procyanidines (OPC), ainsi que leurs mélanges.

La présente invention concerne aussi principalement une utilisation cosmétique de la taurine et/ou de l'hypotaurine et/ou leurs sels acceptables, en association avec au moins un polyphénol et/ou un extrait en comportant, pour une administration par voie orale, pour le traitement de la chevelure ; le dit polyphénol étant choisi parmi une procyanidine, une proanthocyanidine, un mélange de monomères catéchines avec des oligomères de procyanidines (OPC), ainsi que leurs mélanges.

La présente invention concerne aussi principalement une utilisation cosmétique de la taurine et/ou de l'hypotaurine et/ou leurs sels acceptables, en association avec au moins un polyphénol et/ou un extrait en comportant, pour une administration par voie orale, pour le traitement et la prévention du vieillissement du cheveu ; le dit polyphénol étant choisi parmi une procyanidine, une proanthocyanidine, un mélange de monomères catéchines avec des oligomères de procyanidines (OPC), ainsi que leurs mélanges

Aussi, la présence description divulgue l'utilisation de la taurine et/ou de l'hypotaurine dans des compositions orales pour la prévention et le traitement des désordres fonctionnels de l'unité pilo-sébacée et notamment pour la prévention et le traitement de l'alopécie. Elle vise également l'utilisation d'acide(s) gras, de polyphénol et/ou d'extraits en comportant, éventuellement en association avec de la taurine dans des compléments alimentaires pour le traitement et la prévention de ces mêmes désordres.

Certaines altérations physiologiques apparaissent avec l'âge, les variations saisonnières, le stress et les agressions atmosphériques. Il s'agit notamment, de la réduction de la densité des cheveux au cours du vieillissement, le nombre et le diamètre des tiges pilaires diminuant. En particulier certains sujets développent le phénomène d'alopécie.

Pour prévenir les altérations de la chevelure se manifestant principalement avec l'âge, on a jusqu'à présent utilisé des acides aminés essentiels, reconnus indispensables comme nutriment pour la synthèse de la kératine dans le bulbe pileux. Ainsi la méthionine, la cystine et la cystéine sont connues pour avoir un impact direct sur le métabolisme du follicule pileux. Toutefois ces acides aminés essentiels agissent sur la synthèse des protéines qui n'est pas le seul mécanisme intervenant dans le phénomène de l'alopécie.

Parmi les causes de l'alopécie, on a en effet déterminé qu'une altération du tissu conjonctif périfolléculaire se traduisait par une rigidification de la gaine conjonctive qui expliquerait la miniaturisation du follicule pileux, signe de vieillissement de l'unité pilosébacée.

De plus, ces altérations du cheveu sont souvent accompagnées d'altération de l'état du scalp, telles la production abondante de sébum. L'hypersécrétion sébacée ou séborrhée et ses conséquences, l'acné par exemple, se manifestent fréquemment au moment de la puberté, mais peuvent se poursuivre à l'âge adulte, notamment chez les femmes, pour des causes hormonales.

Ces désordres peuvent se produire conjointement, à différents degrés, chez un même individu.

Pour lutter contre le phénomène d'alopécie, qui caractérise le follicule pileux, on a préconisé l'utilisation de médicaments inhibiteurs du métabolisme du collagène. Il est connu d'utiliser notamment le minoxidil, et à ce jour, le mécanisme d'action du minoxidil, connu pour pouvoir lutter contre le processus de miniaturisation du follicule pileux, sans être un anti-androgène, est encore inconnu.

Pour lutter contre l'hypersécrétion sébacée, des traitements par voie locale ont été proposés dont l'isotrétinoine, mais ce traitement n'est pas dénué d'effets secondaires sérieux.

On a également préconisé l'utilisation d'anti-androgènes contre l'alopécie et l'hypersécrétion sébacée, par voie systémique. Ce type de traitement n'est cependant pas dénué d'effets secondaires sérieux en particulier sur les organes sexuels.

Pour sa part, le document WO 99/22728 décrit de nombreux composés dont les acides gras, notamment, pour des utilisations thérapeutiques. Toutefois, les médicaments présentent des inconvénients liés aux risques inhérents à leur utilisation, dans la mesure où les médicaments sont des xénobiotiques. En outre, les médicaments ont en général un spectre d'action très ciblé, alors même que les causes de l'altération de l'unité pilo-sébacée sont multiples.

Par ailleurs, la taurine est décrite comme un activateur cellulaire pour la régulation des cellules capillaires et proposée dans des compositions de stimulants capillaires d'application topique, dans le document WO 02 24189. Néanmoins, la taurine utilisée comme activateur cellulaire par voie topique a des effets limités du fait que la perte d'activité cellulaire peut être due à de multiples causes de l'alopécie. Si ces causes persistent, les effets temporaires d'une application topique d'activateur cellulaire sont limités. De plus, les compositions à usage topique présentent de leur côté les inconvénients liés à l'application locale. La fréquence des applications est généralement plus importante et l'application de ces compositions sur une surface à traiter de taille importante, peut requérir un certain temps.

Ainsi, EP116489A décrit que la taurine peut être utilisée par voie topique pour la dépigmentation, ou en tant qu'agent bioactivateur pour la peau.

WO98/33472A divulgue la taurine comme activateur cellulaire afin de stimuler la pousse des cheveux.

WO00/18736A décrit un produit alimentaire comprenant de la vitamine C, de la taurine, du curcumin et de l'aloe vera, utile pour traiter les désordres de la peau.

US6103272A décrit des compositions utiles entre autre, pour le traitement du vieillissement de la peau ou du derme, tels que l'alopécie.

WO02/096221 décrit une préparation diététique comprenant un mélange d'acides aminés, éventuellement en association avec d'autres composés, tels que d'autres acides aminés ou des vitamines, pour lutter contre la perte des cheveux et améliorer la repousse.

WO97/25972 enseigne des compositions orales pour améliorer la pousse des cheveux, et/ou la structure du cheveu, comprenant des acides aminés (tels que la proline), en association avec de la taurine.

On constate donc qu'il subsiste un besoin d'actifs que l'on peut administrer par voie orale, efficaces dans le traitement et/ou la prévention des signes du vieillissement du cheveu et/ou des désordres fonctionnels de l'unité pilo-sébacée, et notamment de l'alopécie, et dépourvus d'effets secondaires. L'unité pilo-sébacée comporte un follicule pileux et sa glande sébacée.

De façon surprenante, la Demanderesse a mis en évidence d'une part que la taurine a un intérêt pour réguler l'altération du tissu conjonctif du follicule pileux, et peut ainsi être avantageusement utilisée dans le traitement et la prévention du vieillissement de l'unité pilo-sébacée et/ou de l'alopécie. Elle a en effet pu observer que la taurine réduit l'incorporation de proline sans altérer celle de la leucine ; ceci montre l'intérêt de la taurine pour réduire spécifiquement l'accumulation de collagène, sans altérer la synthèse globale des protéines.

D'autre part, la Demanderesse a également constaté que des extraits riches en polyphénols et/ou en acides gras, utilisés par voie orale, notamment à titre de complément alimentaire, ont une activité bénéfique sur les désordres se manifestant au niveau de l'unité pilo-sébacée. Des compositions orales comportant des polyphénols et/ou des acides gras peuvent notamment prévenir l'activation cutanée de la testostérone (intacrine), et l'augmentation de la fonction sébacée qui en résulte, et ceci sans effet général sur l'appareil génital ou les fonctions sexuelles.

Par ailleurs, la Demanderesse a noté que le fait de privilégier une administration par voie orale, permettait d'obtenir un effet anti-chute des cheveux sans induire un effet stimulateur au niveau de la pousse du système pileux autre que le système capillaire. Elle a ainsi constaté qu'une administration par voie orale, des matières actives considérées selon l'invention s'avérait particulièrement efficace pour maintenir une belle chevelure en agissant sur la densité capillaire c'est-à-dire le nombre de cheveux par cm² de cuir chevelu et en réduisant l'hétérogénéité des diamètres capillaires.

Ainsi, la présente description divulgue également l'utilisation de la taurine et/ou de l'hypotaurine et/ou leurs sels acceptables dans une composition orale, pour la préparation d'une composition orale utile dans le traitement et la prévention du vieillissement de l'unité pilo-sébacée et/ou de l'alopécie et en particulier pour prévenir ou réduire l'altération du tissu conjonctif du follicule pileux notamment induite par une rigidification de la gaine conjonctive.

La taurine, l'hypotaurine ou leurs sels acceptables peuvent être mis en œuvre, dans des compositions à usage oral, utiles pour réduire ou prévenir l'altération du follicule pileux induite par une réticulation et/ou une synthèse excessives des collagènes naturels, pour réguler le métabolisme et la structure des collagènes dans le tissu cutané, péri folliculaire, et en particulier dans la gaine conjonctive du follicule pileux. En particulier, la taurine et/ou l'hypotaurine et/ou leurs sels acceptables peuvent être utilisés pour prévenir la miniaturisation du follicule pileux.

Selon une variante, la taurine et/ou hypotaurine et/ou leurs sels acceptables sont utilisés en association avec l'un au moins des composés choisis parmi les acides gras, les polyphénols et les extraits en comportant.

La présente description divulgue également l'utilisation de polyphénol(s) choisi(s) parmi les flavonols, anthocyanines, flavanols, proanthocyanidines et flavanones, et stilbènes et/ou d'acide(s) gras choisi(s) parmi les acides gras polyinsaturés essentiels n-6 et n-3, comportant entre 18 et 22 atomes de carbone, ainsi que leurs esters, et leurs mélanges et/ou d'un extrait en comportant, pour la préparation d'une composition orale, notamment d'un complément alimentaire utile dans le traitement ou la prévention des désordres de l'unité pilo-sébacée, en particulier utile pour réduire ou prévenir la séborrhée voire pour réduire ou prévenir la chute des cheveux.

Les compositions orales décrites sont notamment avantageuses pour réduire ou prévenir le métabolisme excessif des androgènes au niveau de la peau et/ou réduire ou prévenir l'impact de la testostérone sur l'unité pilo-sébacée.

La présente description divulgue également des compositions pour l'absorption orale comportant de la taurine et/ou de l'hypotaurine et/ou de leurs sels acceptables pour l'absorption orale, lesdites compositions comportant à titre d'actif au moins 0,05 % à 80 % en poids de taurine et/ou d'hypotaurine et /ou de leurs sels acceptables et un excipient et étant exemptes de vitamine C et comportant en outre le cas échéant au moins un polyphénol et/ou un acide gras et/ou un de leurs sels acceptables dans une composition orale.

Les polyphénols mentionnés dans la présente description incluent les flavones, flavonols, isoflavones, anthocyanines, flavanols, proanthocyanidines et flavanones, et stilbènes et les acides gras sont choisis parmi les acides gras polyinsaturés essentiels n-6 et n-3, comportant entre 18 et 22 atomes de carbone, ainsi que leurs esters, et leurs mélanges.

Plus particulièrement, les polyphénols selon l'invention sont choisis parmi les procyanidines, les proanthocyanidines, les mélanges de monomères catéchines avec des oligomères de procyanidines (OPC), ainsi que leurs mélanges.

La présente description divulgue également une composition pour l'absorption orale comportant au moins un polyphénol choisi parmi les flavonols, anthocyanines, flavanols, proanthocyanidines et flavanones et stilbènes et/ou un acide gras choisi parmi les acide(s) gras polyinsaturé(s) essentiel(s) n-6 et n-3, comportant entre 18 et 22 atomes de carbone, ainsi que leurs esters, et leurs mélanges et/ou un extrait en comportant, en association avec de la taurine et/ou l'hypotaurine et/ou leurs sels acceptables pour l'absorption orale et le cas échéant un excipient.

Les compositions pour l'absorption orale peuvent comprendre 0,01 à 30 % en poids de taurine, et/ou d'hypotaurine et/ou de leurs sels acceptables, en association avec 0,1 à 50 % en poids d'extraits comportant au moins un polyphénol, et notamment avec 0,1 à 25 % en poids, notamment 0,1 à 20 % en poids, voire 0,1 à 15 % en poids de catéchines.

Les compositions pour l'absorption orale peuvent notamment posséder de la taurine, et/ou hypotaurine et/ou leurs sels acceptables, en association avec des polyphénols dans un rapport pondéral polyphénol/taurine au moins égal à 0,5, en particulier supérieur ou égal à 0,75, notamment supérieur ou égal à 1.

La présente description divulgue également une composition pour l'absorption orale comportant au moins 0,01 à 30 % en poids de taurine, et/ou d'hypotaurine et/ou de leurs sels acceptables, en association avec 0,01 à 10 % en poids d'acides gras.

Selon un mode de réalisation particulier, les compositions orales de l'invention sont des compléments alimentaires.

La présente description divulgue par ailleurs un procédé cosmétique de traitement et de prévention du vieillissement du cheveu et/ou de l'alopécie par l'administration orale de taurine et/ou d'hypotaurine et/ou de sels acceptables pour l'administration orale.

Selon un autre de ses aspects, la présente description divulgue un procédé cosmétique de traitement et de prévention des désordres de l'unité pilo-sébacée par l'administration orale d'au moins un acide gras, un polyphénol ou d'un extrait en comportant éventuellement en association avec de la taurine et/ou de l'hypotaurine et/ou leurs sels acceptables.

Dans un mode de réalisation particulier, on administre la taurine, l'hypotaurine ou leurs sels acceptables à une dose de 0,5 à 4000 mg par jour en équivalent taurine, le ou les acide(s) gras à une dose de 0,5 à 5400 mg/j et/ou le ou les polyphénol(s) à une dose de 0,5 à 2000 mg/j.

Selon l'invention, les effets attendus sont atteints sans les effets indésirables d'un médicament, de façon peu coûteuse par rapport au prix d'un traitement par un médicament. La prise orale du ou des actifs permet un effet plus constant, sans que l'on soit obligé de réitérer les applications.

Plus particulièrement, on lève les limitations d'efficacité quand cette prise orale est réalisée chez des sujets chez qui on a détecté un signe précurseur de désordre fonctionnel de l'unité pilo-sébacée notamment de l'alopécie, comme un état de réticulation excessive du conjonctif périfolliculaire, par exemple par histologie ou par macro-photographie du scalp.

L'invention sera mieux comprise à la lecture de la description détaillée et des exemples suivants.

### LA TAURINE, L'HYPOTAURINE

A titre d'actif selon l'invention, on peut utiliser la taurine et/ou l'hypotaurine, qui en est un métabolite. On peut utiliser également leurs sels acceptables dans de telles compositions orales. Dans la mesure où les compositions selon l'invention sont destinées à être administrées à un sujet, ces sels sont bien entendu choisis pour leur totale innocuité. A ce titre, conviennent tout particulièrement à l'invention les sels alcalins ou alcalinoterreux, en particulier les sels de magnésium, manganèse, fer II ou zinc.

Selon l'invention, l'hypotaurine, ou la taurine sont utilisées à des doses journalières allant de 0,5 à 4000 mg par jour, de préférence 10 à 500 mg par jour. De façon encore préférée, la dose journalière est d'environ 50 à 150 mg par jour. Les doses indiquées dans la présente description sont les doses en équivalent taurine.

### LES POLYPHENOLS

Ces composés sont en général issus de plantes, et leurs structures sont classées selon la nature du squelette hydrocarboné (Laura Bravo Nutrition Review 1998 56 pp 317-333, Scalbert A. Williamson GJ Nutr2000 130 2073s 2085S).

Selon la présente description, on entend plus particulièrement par polyphénols, les composés de type flavonoïdes, c'est à dire les flavones, flavonols, isoflavones, anthocyanines, flavanols, proanthocyanidines et flavanones, et stilbènes ; ce qui inclut en particulier les flavonols, anthocyanines, flavanols, proanthocyanidines et flavanones et stilbènes.

Les principaux flavanols sont les catéchines, gallocatéchines. Les procyanidines sont des polymères de flavonols présents sous formes de mélanges de polymères de faible degré. Ils peuvent être associés à des catéchines dans les extraits végétaux.

Parmi les polyphénols ou mélanges de polyphénols, on peut citer la catéchine, l'épicatéchine, l'épigallocatéchine-3-O-gallate, l'épigallocatéchine, l'épicatéchine-3-gallate, les procyanidines, proanthocyanidines et leurs mélanges.

Selon l'invention, le dit ou les dits polyphénols(s) sont choisis parmi une procyanidine, une proanthocyanidine, un mélange de monomères catéchines avec des oligomères de procyanidines (OPC), ainsi que leurs mélanges.

Il est particulièrement avantageux d'utiliser des monomères catéchines en mélange le cas échéant avec des oligomères procyanidines (OPC). Ainsi, les polyphénols mis en œuvre selon l'invention ne peuvent être constitués que de monomères catéchines.

Ces apports en polyphénols peuvent être faits à partir de composés isolés et/ou à partir d'extraits végétaux et leurs mélanges.

On pourra selon l'invention utiliser des extraits végétaux pouvant apporter l'ensemble de ces polyphénols.

Plus particulièrement, les catéchines sont très abondantes dans le thé (Camellia Simensis), et le raisin (Vitis Vinifera) et autres fruits (pomme, poire, pomme de pin (Pinus Maritima). Les breuvages (vin, bière, thé) et le chocolat (Théobroma Cacao) sont des sources pouvant constituer des apports en catéchines.

Ces polyphénols pourront être utilisés seuls ou utilisés sous forme de mélanges, et peuvent être ingérés sous différentes formes de compléments nutritionnels (dragée, gels, poudres solubles, gélules, capsules, aliments enrichis...).

Ces polyphénols alimentaires peuvent être utilisés à des doses de 0,5 à 2000 mg/jour, notamment de 0,5 à 1000 mg/jour, de préférence de 20 à 300 mg/j.

En particulier, ces polyphénols peuvent être administrés par voie orale à des doses dites nutritionnelles c'est-à-dire équivalentes aux doses absorbées par un sujet humain soumis à un régime alimentaire équilibré.

A titre d'exemple, on peut citer un extrait de pépins de raisin à 40 % OPC, un extrait de vin rouge à 30 % de polyphénols totaux et/ou un extrait de thé vert à 30 % de catéchines.

Les oligomères procyanidines (OPC) peuvent être utilisés à des doses de 0,5 à 1000 mg/j, de préférence 20 à 250 mg/j. Ils peuvent être apportés par un extrait de pépins de raisin, que l'on dose selon son titre OPC. A titre d'exemple, pour un extrait de pépin de raisin à 40 % OPC, ci-dessus, on l'utilise à une dose de 150 mg/j, soit 60 mg/j OPC.

Par ailleurs, les catéchines peuvent être utilisées à des doses de 0,5 à 1000 mg/j, de préférence 20 à 300 mg/j. Elles peuvent être apportées par exemple par un extrait de thé vert à 30 % de catéchines, la dose d'extrait étant alors de l'ordre de 375 mg/j, soit 112,5 mg/j de catéchines.

Comme exemple de doses journalières en polyphénols, on peut citer des doses journalières d'un extrait de vin rouge riche en composants polyphénoliques (600 mg de poudre d'extrait de vin rouge : 12 mg OPC/personne/jour soit 18 mg polyphénols totaux), des doses journalières d'un extrait de pépin de raisin riche en composants polyphénoliques (300 mg de poudre d'extrait de vin rouge : 18 mg OPC/personne/jour soit 27 mg polyphénols totaux), des doses journalières extrait de thé vert riche en composants polyphénoliques (225 mg de poudre d'extrait de Thé vert : 67,5 mg de catéchines/ jour).

Les polyphénols peuvent être choisis dans une des catégories ci-dessus, et on peut aussi opérer des mélanges. Les compositions de compléments alimentaires peuvent comporter 0,01 à 10 % en poids d'au moins un polyphénol.

Dans le cas où ces polyphénols sont administrés en association avec la taurine, et/ ou l'hypotaurine, ils peuvent être administrés à raison de 0,1 à 50 % en poids pour 0,01 à 30 % en poids de taurine et/ou l'hypotaurine et/ou leurs sels acceptables.

La Demanderesse a notamment mis en évidence que l'administration par voie orale d'une dose de 37 mg/J/kg d'un concentré de vin rouge, ce qui équivaut à une dose de 220 mg/J/kg pour un sujet humain de 60 kg, avait un effet efficace sur la chute des cheveux sans manifestation d'effet secondaire indésirable au niveau de la prostate. Ceci est notamment illustré dans les exemples ci-après.

Comme précisé précédemment, il est avantageux d'utiliser les polyphénols en association avec la taurine dans un rapport pondéral polyphénol/taurine au moins égal à 0,5, en particulier supérieur ou égal à 0,75, notamment supérieur ou égal à 1. Plus particulièrement ce rapport pondéral peut varier de 0,5 à 2, notamment de 0,75 à 1,5 voire de 0,9 à 1,3 en particulier être de l'ordre de 1,2.

S'avèrent également tout particulièrement avantageuses des compositions conformes à l'invention comprenant de 0,01 à 30 % en poids de taurine et/ou d'hypotaurine et/ou de leurs sels acceptables et notamment de taurine, en associant avec 0,1 à 25 % en poids de catéchines, notamment 0,1 à 20% en poids de catéchines présentes sous la forme d'un extrait végétal ou d'un mélange d'extraits végétaux.

Dans le cas particulier des catéchines, ces composés peuvent être associés à la taurine, l'hypotaurine et/ou leurs sels dans un rapport pondéral catéchine /taurine au moins égal à 0,4, notamment supérieure à 0,7, voire compris entre 0,7 et 1,5.

### LES ACIDES GRAS

A titre d'acides gras, on entend les acides gras polyinsaturés, c'est-à-dire tous les acides gras avec doubles liaisons interrompues cis, cis-méthylène.

Les acides gras polyinsaturés alimentaires sont définis selon la longueur de la chaîne carbonée et la position de la double liaison. On range actuellement les acides gras essentiels en deux groupes (ω3 et ω6) caractérisés par la position de l'insaturation la plus proche du groupe méthyle terminal.

Conviennent tout particulièrement les acides gras de deux familles d'acides gras polyinsaturés essentiels des familles d'acides gras n-6 et n-3, comportant entre 18 et 22 atomes de carbone ainsi que leurs esters et leurs mélanges. Ces acides gras présentent en effet l'intérêt d'être autorisés selon les normes alimentaires.

De préférence, ces acides gras ne sont pas associés à des terpènes ou dérivés terpéniques.

Pour les acides gras polyinsaturés des séries n-6, appelés « oméga-6 », on peut citer le premier, l'acide linoléique, à 18 atomes de carbone et deux insaturations : (18 :2ω6), et l'acide γ-linolénique (18 :3ω6) est aussi un acide gras particulièrement intéressant selon l'invention.

Les sources d'acide γ-linolénique seront choisies parmi des huiles végétales (huiles d'onagre, de bourrache, de pépin de cassis et de chanvre), les extraits de la spiruline, Spirula. maxima et S. platensis.

Pour les acides gras polyinsaturés des séries n-3, appelés « oméga-3 », le premier est l'acide alpha-linolénique (18 ;3ω3) ; l'acide stéaridonique (C18 :4 n-3) est aussi un acide gras particulièrement intéressant dans l'invention.

Les huiles végétales de noix (Juglans regia) et de soja (Glycina max) par exemple, sont riches en acides gras polyinsaturés de la série oméga 3 au même titre que les huiles de poisson.

Les acides gras polyinsaturés ω3 se retrouvent, via la chaîne alimentaire, dans le zooplanton, les crustacés / mollusques et les poissons.

Les huiles de poissons constituent la principale source industrielle d'EPA (acide eicosapentaénoïque = 20 :5 ω3) et le DHA (acide docosahéxaénoïque = 22 :6 ω3). Cependant, les biomasses de microalgues peuvent aussi constituer une matière première d'extraction des ω3.

La qualité nutritionnelle des microalgues peut être améliorée par un choix judicieux de souches et par une orientation métabolique liée aux conditions de culture.

L'intérêt pour les microalgues est d'autant plus grand qu'elles synthétisent des acides gras, tel l'EPA et le DHA.

De préférence, on utilise l'acide linoléique, l'acide γ-linolénique, l'acide linolénique, l'acide stéaridonique, la crocétine et l'acide 5, 8, 11, 14 éicosatétrainoïque et leurs mélanges ou des extraits les comportant. Ainsi, le ou les acides gras et/ou le ou les extraits peuvent être utilisés seuls ou en mélanges.

Les doses journalières préconisées sont, pour les acides gras comprises entre 0,5 et 3500 mg/j, notamment entre 5 et 1500 mg/j.

Les doses journalières préconisées sont, pour les acides gras n-3 comprises entre 0,5 et 2500 mg/j, de préférence 5 à 360 mg/j, et pour les acides gras n-6 comprises entre 0,5 et 2600 mg/j, de préférence 5 à 1200 mg/j.

Les acides gras peuvent être choisis dans une des catégories ci-dessus, et on peut aussi opérer des mélanges. Les compositions orales peuvent comporter 0,01 à 10 % en poids d'au moins un acide gras.

Dans le cas d'une association avec la taurine et/ou l'hypotaurine et/ou de leurs sels acceptables, et d'au moins un polyphénol choisi parmi une procyanidine, une proanthocyanidine, un mélange de monomères catéchines avec des oligomères de procyanidines (OPC), ainsi que leurs mélanges et/ou un extrait en comportant, les compositions selon l'invention peuvent comprendre de 0,01 à 30 % en poids de taurine et/ou d'hypotaurine et/ou de leurs sels acceptables avec 0,01 à 10 % en poids d'acides gras.

### AUTRE(S) ACTIF(S)

Les actifs considérés, à savoir la taurine, l'hypotaurine ou leurs sels, les acides gras, les polyphénols et leurs mélanges peuvent être associés à un ou plusieurs autres actifs tels que notamment des vitamines et antioxydants, éventuellement sous forme de complexes.

Au sens de la présente invention, le terme actif signifie que le composé considéré, par exemple l'association de taurine et/ou de l'hypotaurine et/ou leurs sels acceptables, en association avec au moins un polyphénol choisi parmi une procyanidine, une proanthocyanidine, un mélange de monomères catéchines avec des oligomères de procyanidines (OPC), ainsi que leurs mélanges, est mis en œuvre pour manifester l'activité biologique et chimique qui lui est propre et non pas pour une fonction de type véhicule ou excipient.

Bien entendu, les compositions selon l'invention peuvent contenir plusieurs actifs.

A titre d'actifs utilisables, on peut citer le zinc et ses sels notamment le sulfate et le glucanate, les vitamines B5, B6, B8, C, E, ou PP, le β-carotène et les caroténoïdes, les extraits d'ail sous forme de sulfure d'allyle ou d'ajoen par exemple, le sélénium, la curcumine, les curcuminoïdes, la niacine, l'acide lithospermique, et l'adénosine.

En particulier, on peut utiliser un complexe anti-oxydant comprenant les vitamines C et E, du zinc ou ses sels, le sélénium et au moins un caroténoïde, notamment le caroténoïde choisi parmi le β-carotène, le lycopène, la zéaxanthine et la lutéine.

On préfère un complexe anti-oxydant comportant par exemple de 100 à 150 mg de vitamine C pour 80 à 120 µg de Sélénium, 20 à 50 mg de vitamine E, 10 à 40 mg de zinc et 3 à 10 mg de β-carotène.

Toutefois, les compositions selon l'invention peuvent avantageusement contenir moins de 1% en poids de vitamine C, voire en être exempte.

Les actifs selon l'invention peuvent en outre être associés à des actifs anti-chute connus, et notamment des composés améliorant encore leur activité sur la repousse et/ou sur le freinage de la chute des cheveux, tels que plus particulièrement les composés suivants :
- les esters d'acide nicotinique, dont plus particulièrement les nicotinates d'alkyle en C3-C6 et notamment le nicotinate de méthyle ou d'hexyle, le nicotinate de benzyle ou de tocophérol ;
- les agents anti-inflammatoires stéroïdiens et non stéroïdiens bien connus dans l'état de la technique et en particulier l'hydrocortisone, ses sels et ses dérivés, l'acide niflumique ;
- les rétinoïdes et plus particulièrement l'acide t-trans rétinoïque appelé encore trétinoïne, l'isotrétinoïne, le rétinol ou la vitamine A et ses dérivés, tels que l'acétate, le palmitate ou le propionate, le motrétinide, l'étrétinate, le t-rans rétinoate de zinc ;
- les agents antibactériens choisis plus particulièrement parmi les macrolides, les pyranosides et les tétracyclines et notamment l'érythromycine ;
- les agents antagonistes de calcium tels que la Cinnarizine et le Diltiazem ;
- des hormones, telles que l'estriol ou des analogues ou la thyroxine et ses sels ;
- des agents antiandrogènes, tels que l'oxendolone, spironolactone, le diéthylstilbestrol ;
- des capteurs de radicaux OH, tels que le diméthylsulfoxyde ;
- des oligosaccharides estérifiés, tels que ceux décrits dans EP-A-0 211 610 et EP-A-0 064 012 ;
- des dérivés d'acide hexosacchariques, tels que ceux décrits dans EP-A-0 375 388, en particulier l'acide glucosaccharique ;
- des inhibiteurs de glycosidase, tels que ceux décrits dans EP-A-0 334 586, en particulier le D-glucaro 1,5-lactam ;
- des inhibiteurs de glycosaminoglycanases et protéoglycanases, tels que ceux cités dans EP-A-0 277 428, en particulier la L-galactono 1,4-lactone ;
- des inhibiteurs de tyrosine kinase, tels que ceux décrits dans EP-A-0 403 238, en particulier le 1-amido 1-cyano-(3,4-dihydroxyphényl)éthylène.

On peut également associer avec les actifs de l'invention, éventuellement en mélange avec les autres, des composés tels que le Diazoxyde correspondant au méthyl-3 chloro-7[2H]benzothiadiazine-1,2,4 dioxyde-1-1 ; la Spiroxazone ou 7-(acétylthio)-4',5'-dihydrospiro-[androst 4-ène- 17,2'-(3'H)furan]3 one ; des phospholipides tels que la lécithine ; l'acide salicylique et ses dérivés décrits plus particulièrement dans le brevet français n° 2 581 542, et plus particulièrement les dérivés d'acide salicylique porteurs d'un groupement alcanoyle ayant 2 à 12 atomes de carbone en position 5 du cycle benzénique, des acides hydroxycarboxyliques ou cétocarboxyliques et leurs esters, des lactones et leurs sels correspondants; l'anthraline, les acides eicosatriynoïque-5,8,11, leurs esters et amides, le minoxidil et ses dérivés, composés décrits dans EP 353 123, EP 356 271, EP 408 442, EP 522 964, EP 420 707, EP 459 890, EP 519 819, US 4 139 619 et US 459 812.

Les composés ci-dessus sont incorporés dans les compositions orales et notamment les compléments alimentaires pour autant que leur utilisation en tant que complément alimentaire soit possible, et leur formulation compatible avec celle des actifs de l'invention. Ces actifs additionnels sont utilisés selon l'invention à des doses compatibles avec leur usage en tant que compléments alimentaires. Ainsi, pour certains composés, on préférera les utiliser par voie topique, en complément des compléments alimentaires de l'invention.

Pour l'ingestion de l'actif ou des actifs, de nombreuses formes de réalisation de compositions orales et notamment de compléments alimentaires sont possibles. Leur formulation est réalisée par les procédés usuels pour produire des dragées, gélules, gels, émulsions, comprimés, capsules ou solutions liquides notamment ampoules buvables, par exemple. En particulier, le(s) actif(s) selon l'invention peuvent être incorporés dans toutes autres formes de compléments alimentaires ou d'aliments enrichis, par exemple des barres alimentaires, ou des poudres compactées ou non. Les poudres peuvent être diluables à l'eau, dans du soda, des produits laitiers ou dérivés du soja, ou être incorporées dans des barres alimentaires.

Les actifs peuvent être formulés avec les excipients et composants usuels pour de tels compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires sont connus dans ce domaine et ne font pas ici l'objet d'une description détaillée.

Un procédé cosmétique selon l'invention est mis en œuvre par une prise orale, journalière par exemple, de composition orale ou complément alimentaire qui peuvent être par exemple sous forme de gélules, gels, dragées, émulsions, comprimés, capsules ou ampoules buvables, en quantité et nombre adéquats, selon leur forme, pour que la taurine et/ou l'hypotaurine ou leurs sels acceptables soient ingérés à raison de 0,5 à 4000 mg par jour, de façon préférée 10 à 500 mg par jour, et de façon plus préférée environ 150 mg par jour, en équivalent taurine et/ou que le(s) polyphénol(s) soient ingérés à des doses de l'ordre de 0,5 à 2000 mg/j, et/ou que les acides gras soient ingérés à des doses de 0,5 à 5400mg par jour, préférentiellement de 5 à 1600mg par jour.

Un procédé selon l'invention peut consister en une prise unique mais généralement est appliqué sur une durée prolongée d'au moins 4 semaines, voire 4 à 8 semaines, avec le cas échéant une ou plusieurs périodes d'interruption.

A titre d'exemple, pour l'acide gamma-linolénique, qui peut être apporté par de l'huile de pépin de cassis, on peut prévoir des doses de l'ordre de 10 à 3000 mg/j, de préférence de 50 à 1000mg/j.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les ingrédients sont mélangés, avant leur mise en forme, dans l'ordre et dans les conditions facilement mises en œuvre par l'homme de l'art.

Les exemples et figures soumis ci-après sont présentés à titre illustratif et non limitatif du domaine de l'invention.
Figure 1 : Elle représente les résultats de l'ingestion d'acides gras par des hamsters, dans un test CVO.
Figure 2 : Elle représente les résultats de l'ingestion de polyphénols par des hamsters dans un test CVO.
Figure 3 : Elle présente les résultats d'antichute de cheveux constatés chez des sujets traités selon l'invention et des sujets témoins.

### EXEMPLE DE MISE EN EVIDENCE DE L'ACTIVITE DES ACIDES GRAS, ET DES POLYPHENOLS.

Afin de mettre en évidence l'activité de ces composés un test de détection d'activité au niveau d'une formation pilosébacée spécifique a été utilisé : le test du CVO.

Le CVO (organe costo-vertébral) du Hamster est une région cutanée riche en unités pilosébacées (follicules pileux et leurs glandes sébacées). La taille de cette formation est augmentée sous l'action de la testostérone. Le test (Liao S & al. Arch Dermatot Res 2001 Apr :293(4) :200-205) consiste à déterminer l'action anti-androgène de composés sur le CVO, c'est-à-dire à déterminer si les composés empêchent l'action de la testostérone.

### 1/Test CVO avec DES ACIDES GRAS (référence):

Dans ce test, l'huile de pépin de cassis à 10 % dans l'aliment durant 85 jours est donnée à des Hamsters mâles. On constate que ce complément nutritionnel prévient l'augmentation de la taille du CVO induite par la testostérone.

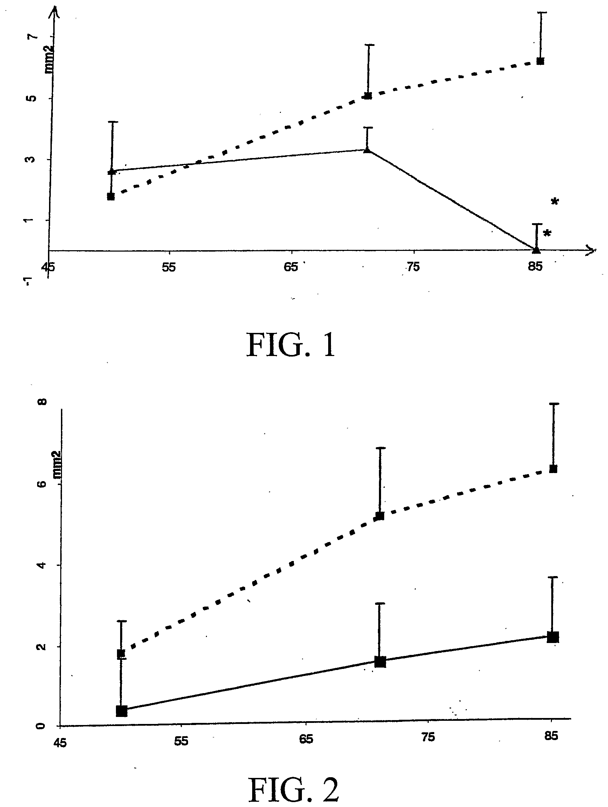

Ainsi, à la figure 1, la courbe en pointillé représente l'évolution de la taille du CVO (mm2) chez des animaux témoins, sans supplémentation, au cours de l'expérience (jours en abscisse): il y a augmentation de la taille du CVO induite par la testostérone. La courbe en trait continu représente l'évolution de la taille du CVO chez des animaux avec supplémentation : cette augmentation est réduite à pratiquement zéro.

### 2/TEST CVO avec DES POLYPHENOLS (référence):

On a fait ingérer à des hamsters un nutriment sous forme de concentré de vin rouge Robertet comportant 18 % de composants flavanoïdes (0,11 g dans 43 g d'aliment) quotidiennement, et on a constaté que la différence CVO droit CVO gauche est pratiquement nulle, à 85 j d'ingestion (figure 2).

Ainsi, à la figure 2, la courbe en pointillé représente l'évolution de la taille du CVO (mm2) chez des animaux témoins, sans supplémentation, au cours de l'expérience (jours en abscisse) : il y a augmentation de la taille du CVO induite par la testostérone. La courbe en trait continu représente l'évolution de la taille du CVO chez des animaux avec supplémentation : cette augmentation est réduite à pratiquement zéro.

### 3/ Absence d'effet secondaire sur la sphère sexuelle (référence):

Chez les hamsters ayant reçu ces compléments nutritionnels par voie orale, on a constaté la répercussion de l'action anti-androgène non seulement sur le CVO, mais également sur les organes sexuels chez des animaux mâles. Dans les deux cas, on a constaté que ces compléments nutritionnels n'altèrent pas le poids des vésicules séminales et de la prostate.

### EXEMPLE DE MISE EN EVIDENCE DE L'ACTIVITE DE LA TAURINE (référence)

On a réalisé une étude dans le but d'évaluer, par une méthode de screening, les effets des composés sur la croissance des fibroblastes et la synthèse des constituants majeurs de la matrice extracellulaire. La technique a permis d'étudier et d'évaluer l'intérêt de la taurine sur ce métabolisme cellulaire. La technique a permis d'étudier et d'évaluer l'intérêt de la taurine sur ce métabolisme cellulaire (T. Shigematsu et al. Biochimica et Biophysica Acta 1200 (1994) 79-83).

Un pool de fibroblastes dermiques humains normaux (NHDF pool PF2, utilisé au huitième passage) a été cultivé en conditions standards dans un milieu : DMEM, L-glutamine 2mM, pénicilline/streptomicyne 50 Ul/ml/50g/ml, sérum de veau fœtal à 0,5 %.

La taurine a été testée à des concentrations de 10 mM et 1 mM, dans un milieu de culture stérile, contre un témoin contrôle non traité.

Les résultats de l'incorporation aux fibroblastes de la thymidine, la proline et la leucine apparaissent dans le tableau I suivant qui présente l'effet de la taurine sur l'incorporation de thymidine, de proline et de leucine dans les macromolécules néosynthétisées par les NHDF en culture in vitro. Les chiffres en gras sont ceux pour lesquels il y a variation significative (sign. stat. pour significativité statistique : p < 0,005). Les résultats sont exprimés en pourcentage du contrôle.

**Tableau 1**

| | Thymidine | | Proline | | Leucine | |
|---|---|---|---|---|---|---|
| | % contrôle | sign.stat | % contrôle | sign.stat | % contrôle | sign.stat |
| Taurine 1 mM | 89 | p>0.05 | 88 | p<0.01 | 100 | p>0.05 |
| Taurine 10 mM | 90 | p>0.05 | 87 | p<0.01 | 105 | p>0.05 |

Il apparaît que la taurine, aux deux concentrations traitées, n'a pas modifié de façon significative l'incorporation par les fibroblastes de la thymidine, représentative de la prolifération cellulaire ni de la leucine, représentative de la synthèse de protéine non collagénique ; en revanche, la taurine a inhibé l'incorporation par les fibroblastes de la proline, de manière significative.

### EXEMPLES DE FORMULATION.

### Exemple 1 (référence)

**FORMULATION DE TYPE DRAGEE**

| | **mg/dragée** |
|---|---|
| Taurine | 50 |
| | |

| **Excipent du noyau de la dragée** | |
|---|---|
| Cellulose microcristalline | 70 |
| Encompress ™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 2 à 3 fois par jour.

### Exemple 2 (référence)

**GELULE GELATINE VEGETALE OU ANIMALE**

| **Principe actif** | **mg/dragée** |
|---|---|
| Taurine | 80 |
| Amidon | 128 |
| Stéarate de magnésium | 2,5 |

Ce type de gélule peut être pris deux ou trois fois par jour.

### Exemple 3 (référence)

**GEL UNIDOSE**

| **Principe actif** | **% pds** |
|---|---|
| Taurine | 4 |
| Levure enrichie en zinc (22,75 % Zn) | 2 |
| | |

| **Excipient** | |
|---|---|
| Rhodigel™ | 2,3 |
| Extrait Cacao | 20 |
| Sorbate de potassium | 0,05 |
| Benzoate de sodium | 0,05 |
| Eau | QSP 100 |

On peut utiliser 200 à 400 ml de ce produit par jour.

### Exemple 4

**GEL UNIDOSE**

| **Principe actif** | **% pds** |
|---|---|
| Taurine | 4 |
| Huile de pépin de cassis | 10 |
| | |

| **Excipient** | |
|---|---|
| Sirop de sucre | 50 |
| Maltodextrine | 17 |
| Gomme Xanthane | 0,8 |
| Benzoate de sodium | 0,2 |
| Eau | QSP 100 |

On peut utiliser 200 à 400 ml de ce produit par jour.

### Exemple 5

**GEL UNIDOSE**

| **Principe actif** | **% pds** |
|---|---|
| Taurine | 4 |
| Huile de pépin de cassis | 10 |
| Complexe antioxydant | * |

| **Excipient** | |
|---|---|
| Sirop de sucre | 50 |
| Maltodextrine | 17 |
| Gomme Xanthane | 0,8 |
| Benzoate de sodium | 0,2 |
| Eau | QSP 100 |

| | |
|---|---|
| * Le complexe antioxydant comporte 120 mg de vitamine C, 100 µg de sélénium, 30 mg de vitamine E, 20 mg de zinc et 6 mg de β-carotène pour 200 ml de gel. | |

On peut utiliser 200 à 400 ml de ce produit par jour.

### Exemple 6

**CAPSULE**

| | **mg/capsule** |
|---|---|
| Taurine | 50 |
| Zinc gluconate | 160 |
| Extrait vin (20 % OPC) | 300 |
| Glycérine | 150 |
| stéarate de magnésium | 0,02 |
| Eau | QSP 900 mg |

### Exemple 7

**CAPSULE**

| | **mg/capsule** |
|---|---|
| Taurine | 50 |
| Zinc gluconate | 160 |
| Extrait vin (20 % OPC) | 300 |
| Glycérine | 150 |
| stéarate de magnésium | 0,02 |
| Complexe vitaminique | QSP ? |
| Eau | QSP 900 mg |

| | |
|---|---|
| * Le complexe vitaminique comporte, 60 mg de vitamine C, 50 µg de sélénium, 15 mg de vitamine E, 10 mg de zinc et 3 mg de lycopène. | |

### Exemple 8

**FORMULATION DE TYPE DRAGEE**

| | **mg/dragée** |
|---|---|
| Taurine | 50 |
| Extraits pépins de raisin (40 % OPC) | 100 |
| Extraits Thé vert (30 % Catechines) | 125 |
| Zinc Sulfate (22,75 %) | 22 |
| | |

| **Excipient du noyau de la dragée** | |
|---|---|
| Cellulose microcristalline | 70 |
| Encompress ™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 3 fois par jour.

### Exemple 9

**FORMULATION DE TYPE DRAGEE**

| | **mg/dragée** |
|---|---|
| Extraits pépins de raisin (40 % OPC) | 100 |
| Extraits thé vert (30 % Catéchines) | 125 |
| Zinc Sulfate (22,75 %) | 22 |
| | |

| **Excipient du noyau de la dragée** | |
|---|---|
| Cellulose microcristalline | 70 |
| Encompress ™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 2 fois par jour.

### Exemple 10

**FORMULATION DE TYPE DRAGEE**

| | **mg/dragée** |
|---|---|
| Taurine | 50 |
| Extraits pépins de raisin (40 % OPC) | 50 |
| Extraits thé vert (30 % Catéchines) | 125 |
| Zinc Sulfate (22,75 %) | 22 |
| | |

| **Excipient du noyau de la dragée** | |
|---|---|
| Cellulose microcristalline | 70 |
| Encompress ™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 3 fois par jour.

### Exemple 11 (référence)

**GELULE GELATINE VEGETALE OU ANIMALE**

| **Principe actif** | **mg/gélule** |
|---|---|
| Extrait pépins de raisin (40 % OPC) | 50 |
| Extrait thé vert (30 % Catéchines) | 175 |
| Amidon | 128 |
| Stéarate de magnésium | 2,5 |

On peut prendre une à quatre gélules par jour.

### Exemple 12

**GELULE GELATINE VEGETALE OU ANIMALE**

| **Principe actif** | **mg/gélule** |
|---|---|
| Taurine | 80 |
| Extrait pépins de raisin (40 % OPC) | 50 |
| Extrait thé vert (30 % Catéchines) | 175 |
| Amidon | 128 |
| Stéarate de magnésium | 2,5 |

On peut prendre une à quatre gélules par jour.

### Exemple 13

**GEL UNIDOSE**

| **Principe actif** | **% pds** |
|---|---|
| Taurine | 4 |
| Extraits pépins de raisin (40 % OPC) | 4 |
| Extrait thé vert (30 % Catéchines) | 6 |
| Levure enrichie en zinc (22,75 % Zn) | 2 |
| | |

| **Excipient** | |
|---|---|
| Rhodigel™ | 2,3 |
| Extrait Cacao | 20 |
| Sorbate de potassium | 0,05 |
| Benzoate de sodium | 0,05 |
| Eau | QSP 100 |

On prend 200 à 400 ml de gel par jour.

### Exemple 14 (référence)

**GEL UNIDOSE**

| **Principe actif** | **% pds** |
|---|---|
| Extrait pépins de raisin (40 % OPC) | 4 |
| Extrait thé vert (30 % Catéchines) | 10 |
| Huile de pépin de cassis | 10 |
| | |

| **Excipient** | |
|---|---|
| Sirop de sucre | 50 |
| Maltodextrine | 17 |
| Gomme Xanthane | 0,8 |
| Benzoate de sodium | 0,2 |
| Eau | QSP 100 |

On prend 200 à 400 ml de gel par jour.

### Exemple 15

**CAPSULE**

| | **mg/capsule** |
|---|---|
| Taurine | 50 |
| Zinc gluconate | 60 |
| Extrait vin (20 % OPC) | 300 |
| Glycérine | 150 |
| stéarate de magnésium | 0,02 |
| Eau | QSP 900 mg |

On prend une à quatre capsules par jour.

### Exemple 16

### POUDRES

Un extrait de vin 1,8 g apportant jusqu'à 540 mg de polyphénols totaux ((dont 360 mg d'OPC)), jusqu'à 120 mg de taurine, 0,01 g édulcorant Goldblend, 0,4 g Arôme FRAM0584 et maltodrextrine 4 g a été utilisé sous forme de poudre à diluer dans l'eau, dans un produit laitier ou incorporé dans une barre alimentaire céréales / fruits à consommer chaque jour.

On a utilisé dans les mêmes conditions :
- Un mélange d'extraits de Vitis Vinefera et/ou d'un de ses produits de biotechnologie (jus raisin, vin...) apportant la même quantité de polyphénols totaux en association avec une source de protéines naturelles riches en taurine apportant une quantité de taurine de 150mg/j.
- Un extrait de Camellia Simensis ou de Théobroma Cacao apportant la même quantité de polyphénols totaux, en association avec une source de protéines naturelles riche en taurine apportant la même quantité de taurine.

### Exemple 17

On adjoint à la formulation gel de l'exemple 13 un complexe vitaminique comportant 120 mg de vitamine C, 100 µg de vitamine E, 20 mg de zinc et 6 mg de β-carotène, dans 200 ml de gel de l'exemple 5 et 60 mg de vitamine C, 50 µg de vitamine E, 10 mg de zinc et 3 mg de β-carotène dans une dragée de l'exemple 10.

### Exemple 18

Dans les formulations de l'exemple 17, on remplace le β-carotène par le lycopène.

### Exemple 19

**GEL UNIDOSE**

| **Principe actif** | **% pds** |
|---|---|
| Taurine | 4 |
| Extraits pépins de raisins à 40 % OPC | 4 |
| Extrait de thé vert à 30 % Catéchines | 6 |
| Huile de pépins de cassis | 10 |
| | |

| **Excipient** | |
|---|---|
| Sirop de sucre | 50 |
| Maltodextrine | 17 |
| Gomme Xanthane | 0,8 |
| Benzoate de sodium | 0,2 |
| Eau | QSP 100 |

On peut prendre une dose de 200 à 400 ml par jour.

### Exemple 20

**CAPSULE**

| | **mg/capsule** |
|---|---|
| Taurine | 50 |
| Zinc gluconate | 60 |
| Extrait vin (20 % OPC) | 200 |
| Huile de pépins de cassis | 300 |
| Glycérine | 150 |
| stéarate de magnésium | 0,02 |
| arôme naturel | |
| Eau | QSP 900 mg |

On peut prendre une à trois de ces capsules par jour.

### Exemple 21

**FORMULATION DE TYPE DRAGEE**

| | **mg/dragée** |
|---|---|
| Taurine | 50 |
| Extraits pépins de raisin (40 % OPC) | 50 |
| Extraits thé vert (30 % Catéchines) | 125 |
| Zinc Sulfate (22,75 %) | 22 |
| | |

| **Excipient du noyau de la dragée** | |
|---|---|
| Cellulose microcristalline | 70 |
| Encompress ™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 3 fois par jour.

### Exemple 22

On adjoint à la formulation de l'exemple 19, un complexe vitaminique comportant 120 mg de vitamine C, 100 µg de vitamine E, 20 mg de zinc et 6 mg de β-carotène, pour 200 ml de gel.

### Exemple 23

On adjoint à la formulation de l'exemple 19, un complexe vitaminique comportant 120 mg de vitamine C, 100 µg de vitamine E, 20 mg de zinc et 6 mg de lycopène pour 200 ml de gel.

### Exemple 24

On adjoint à la formulation de l'exemple 22, un complexe vitaminique comportant 60 mg de vitamine C, 50 µg de vitamine E, 10 mg de zinc et 3 mg de lycopène pour une dragée.

### Exemple 25

**FORMULATION DE TYPE COMPRIME**

| **Principe actif** | **Mg/Comprimé** |
|---|---|
| Taurine | 75 |
| Extraits pépins de raisin (40 % OPC) | 75 |
| Extraits thé vert (30 % Catéchines) | 187,5 |
| Zinc gluconate (14,3% de zinc) | 52,3 |
| | |
| **Excipient** | **Qsp pour 1g** |

Ce type de comprimé est pris 2 fois par jour.

### Exemple 26

Deux groupes de 36 femmes âgées de 18 à 40 ans environ ayant les cheveux fins, mous et séborrhéiques ont absorbé pendant 6 mois :
- soit la formule capillaire de composition comme suit :

| | mg |
|---|---|
| Taurine | 150 |
| Extrait de thé vert à (30 % Catéchines) | 375 |
| Extrait de pépins de raisins (40 % OPC et 20 % catéchines) | 150 |
| Sulfate de zinc (22,75 %) | 15* |

| | |
|---|---|
| **exprimé en poids de zinc* | |

- soit un placébo, comprimé d'aspect identique à base de maltodextrine.

L'effet du traitement est examiné par auto-évaluation, et en appliquant trois coups de peigne à T0, T3 mois et T6 mois.

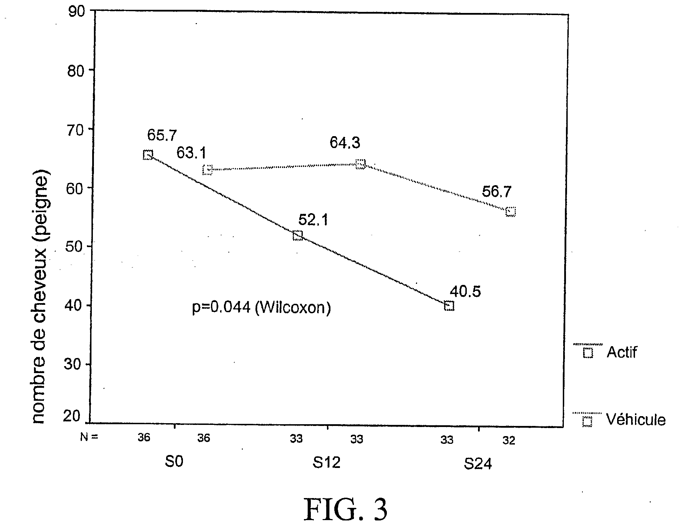

On note une diminution régulière du nombre de cheveux sur le peigne dans le groupe traité, cette différence étant statistiquement significative, comparativement au groupe à 6 mois ayant reçu le placebo. Les résultats sont présentés en figure 3.

## Revendications

1. Utilisation cosmétique de la taurine et/ou de l'hypotaurine et/ou leurs sels acceptables, en association avec au moins un polyphénol et/ou un extrait en comportant, pour une administration par voie orale, pour maintenir une belle chevelure en agissant sur la densité capillaire et en réduisant l'hétérogénéité des diamètres capillaires ;
le dit polyphénol étant choisi parmi une procyanidine, une proanthocyanidine, un mélange de monomères catéchines avec des oligomères de procyanidines (OPC), ainsi que leurs mélanges.

2. Utilisation selon la revendication 1, **caractérisée en ce que** la dose journalière de taurine et/ou d'hypotaurine et/ou leurs sels acceptables, en équivalent taurine est comprise entre 0,5 et 4000 mg/j.

3. Utilisation selon l'une des revendications précédentes, **caractérisée en ce que** la dose journalière de taurine et/ou d'hypotaurine et/ou leurs sels acceptables, en équivalent taurine, est comprise entre 10 et 500 mg/j.

4. Utilisation selon l'une des revendications précédentes, **caractérisée en ce que** la dose journalière en taurine et/ou hypotaurine et/ou leurs sels acceptables, en équivalent taurine, est de 50 à 150 mg/j.

5. Utilisation selon l'une des revendications précédentes, **caractérisée en ce que** les sels acceptables de la taurine et/ou de l'hypotaurine sont les sels de magnésium, manganèse, fer II ou zinc.

6. Utilisation selon l'une des revendications précédentes **caractérisée en ce que** la taurine et/ou l'hypotaurine et/ou leurs sels acceptables sont utilisés en association avec au moins un acide gras, et/ou un extrait en comportant.

7. Utilisation selon la revendication 6, **caractérisée en ce que** les acides gras sont choisis parmi les acides gras polyinsaturés essentiels n-6 et n-3, comportant entre 18 et 22 atomes de carbone, ainsi que leurs esters, et leurs mélanges.

8. Utilisation selon l'une des revendications précédentes, **caractérisée en ce que** la composition se présente sous la forme d'un complément alimentaire.

9. Utilisation selon l'une des revendications 6 à 8, **caractérisée en ce que** les polyphénols sont choisis parmi les procyanidines, proanthocyanidines et leurs mélanges.

10. Utilisation selon l'une des revendications 6 à 9, **caractérisée en ce que** la dose journalière de polyphénol(s), est comprise entre 0,5 et 2000 mg/j.

11. Utilisation selon l'une des revendications 6 à 10, **caractérisée en ce que** la dose journalière de polyphénols est de l'ordre de 0,5 à 1000 mg/j.

12. Utilisation selon l'une des revendications 6 à 11, **caractérisée en ce que** le ou les acides gras sont choisis parmi l'acide linoléique, l'acide γ-linolénique, l'acide linolénique, l'acide stéaridonique, la crocétine et l'acide 5, 8, 11, 14 éicosatétrainoïque et leurs mélanges.

13. Utilisation selon l'une des revendications 6 à 12, **caractérisée en ce que** la dose journalière d'acides gras, est comprise entre 0,5 et 3500 mg/j, de préférence entre 5 et 1500 mg/j.

14. Utilisation selon l'une des revendications 6 à 13, **caractérisée en ce que** la dose d'acides gras n-6 est comprise entre 0,5 et 2600 mg/j, de préférence 5 à 1200 mg/j.

15. Utilisation selon l'une des revendications 6 à 14, **caractérisée en ce que** la dose d'acides gras n-3 est comprise entre 0,5 et 2500 mg/j, de préférence 5 à 360 mg/j.

16. Utilisation selon l'une des revendications précédentes, **caractérisée en ce que** la composition orale comporte en outre l'un au moins des compléments choisis parmi les vitamines C et E, du zinc ou ses sels, le sélénium et au moins un caroténoïde, de préférence un caroténoïde choisi parmi le β-carotène, le lycopène, la zéaxanthine et la lutéine.

17. Composition pour l'absorption orale **caractérisée en ce qu'**elle comprend au moins un polyphénol choisi parmi une procyanidine, une proanthocyanidine, un mélange de monomères catéchines avec des oligomères de procyanidines (OPC), ainsi que leurs mélanges et/ou un extrait en comportant ; en association avec de la taurine et/ou l'hypotaurine et/ou leurs sels acceptables.

18. Composition selon la revendication précédente, **caractérisée en ce qu'**elle comporte à titre d'actif au moins 0,05 à 80 % en poids de taurine et/ou d'hypotaurine et/ou de leurs sels acceptables, en association avec au moins un polyphénol ou un extrait en comportant, pour l'absorption orale et un excipient, ladite composition étant exempte de vitamine C.

19. Composition selon la revendication 17, **caractérisée en ce qu'**elle comporte au moins un acide gras et/ou un de ses sels acceptables.

20. Composition selon la revendication précédente, **caractérisée en ce qu'**elle comprend de 0,01 à 10 % en poids d'au moins un polyphénol et/ou 0,1 à 10 % en poids d'au moins un acide gras.

21. Composition selon la revendication 17 **caractérisée en ce qu'**elle comprend au moins des polyphénols en association avec de la taurine et/ou l'hypotaurine et/ou leurs sels acceptables dans un rapport pondéral polyphénol/taurine au moins égal à 0,5, en particulier supérieur ou égal à 0,75, notamment supérieur ou égal à 1.

22. Composition selon la revendication 17, **caractérisée en ce qu'**elle comprend 0,01 à 30 % en poids de taurine, et/ou d'hypotaurine et/ou de leurs sels acceptables, en association avec 0,1 à 50 % en poids d'extraits comportant au moins un polyphénol.

23. Composition selon la revendication 17 **caractérisée en ce qu'**elle comprend au moins des catéchines en association avec de la taurine et/ou l'hypotaurine et/ou leurs sels acceptables.

24. Composition selon la revendication 23, **caractérisée en ce qu'**elle comprend 0,01 à 30 % en poids de taurine, et/ou d'hypotaurine et/ou de leurs sels acceptables, en association avec 0,1 à 25 % en poids de catéchines.

25. Composition selon la revendication 17, **caractérisée en ce qu'**elle comporte en outre l'un au moins des composés choisis parmi les vitamines C et E, du zinc ou ses sels, le sélénium et au moins un caroténoïde, de préférence un caroténoïde choisi parmi le β-carotène, le lycopène, la zéaxanthine et la lutéine.

26. Composition selon la revendication 17, **caractérisée en ce que** l'excipient est acceptable pour un complément alimentaire sous forme de dragée, gélule, gel, émulsion, comprimé, capsule, ampoule buvable, poudre diluable ou non, barres alimentaires ou aliments enrichis.

27. Composition selon la revendication 17, **caractérisée en ce qu'**il s'agit d'un complément alimentaire.

28. Association de taurine et/ou d'hypotaurine et/ou de sels acceptables, avec au moins un polyphénol et/ou un extrait en comportant, destinée à une utilisation pour administration orale dans le traitement et la prévention de l'alopécie ;
le dit polyphénol étant choisi parmi une procyanidine, une proanthocyanidine, un mélange de monomères catéchines avec des oligomères de procyanidines (OPC), ainsi que leurs mélanges.

29. Association destinée à une utilisation selon la revendication 28 ; dans laquelle la taurine, l'hypotaurine ou leurs sels acceptables sont administrables à une dose de 0,5 à 4000 mg par jour en équivalent taurine.

30. Association destinée à une utilisation selon la revendication 28 ou 29 ; dans laquelle la taurine, l'hypotaurine et/ou leurs sels acceptables sont utilisés en association avec l'un au moins des compléments choisis parmi le zinc, les acides gras, la vitamine C, la vitamine E, un (ou des) caroténoïdes, par exemple le β-carotène ou le lycopène.

31. Utilisation cosmétique de la taurine et/ou de l'hypotaurine et/ou leurs sels acceptables, en association avec au moins un polyphénol et/ou un extrait en comportant, pour une administration par voie orale, pour le traitement de la chevelure ;
le dit polyphénol étant choisi parmi une procyanidine, une proanthocyanidine, un mélange de monomères catéchines avec des oligomères de procyanidines (OPC), ainsi que leurs mélanges.

32. Utilisation cosmétique de la taurine et/ou de l'hypotaurine et/ou leurs sels acceptables, en association avec au moins un polyphénol et/ou un extrait en comportant, pour une administration par voie orale, pour le traitement et la prévention du vieillissement du cheveu ;
le dit polyphénol étant choisi parmi une procyanidine, une proanthocyanidine, un mélange de monomères catéchines avec des oligomères de procyanidines (OPC), ainsi que leurs mélanges.

## Patentansprüche

1. Kosmetische Verwendung von Taurin und/oder von Hypotaurin und/oder deren unbedenklichen Salzen, in Kombination mit mindestens einem Polyphenol und/oder mit einem Extrakt, der solche aufweist, zur Verabreichung auf oralem Wege, um die Schönheit des Haars aufrechtzuerhalten, indem auf die Haardichte eingewirkt wird und indem die Schwankungsbreite der Haardurchmesser verringert wird;
wobei das Polyphenol aus einem Procyanidin, einem Proanthocyanidin, einer Mischung von Catechinmonomeren mit Oligomeren von Procyanidinen (OPC) sowie aus deren Mischungen ausgewählt ist.

2. Verwendung nach Anspruch 1, **dadurch gekennzeichnet, dass** die Tagesdosis an Taurin und/oder an Hypotaurin, und/oder deren unbedenklichen Salzen als Taurin-Äquivalent im Bereich von 0,5 bis 4.000 mg/Tag liegt.

3. Verwendung nach einem der vorhergehenden Ansprüche, **dadurch gekennzeichnet, dass** die Tagesdosis an Taurin und/oder an Hypotaurin, und/oder deren unbedenklichen Salzen, als Taurin-Äquivalent im Bereich von 10 bis 500 mg/Tag liegt.

4. Verwendung nach einem der vorhergehenden Ansprüche, **dadurch gekennzeichnet, dass** die Tagesdosis an Taurin und/oder Hypotaurin, und/oder deren unbedenklichen Salzen, als Taurin-Äquivalent im Bereich von 50 bis 150 mg/Tag liegt.

5. Verwendung nach einem der vorhergehenden Ansprüche, **dadurch gekennzeichnet, dass** es sich bei den unbedenklichen Salzen von Taurin und/oder von Hypotaurin um Magnesium-, Mangan-, Eisen(II)- oder Zinksalze handelt.

6. Verwendung nach einem der vorhergehenden Ansprüche, **dadurch gekennzeichnet, dass** das Taurin und/oder das Hypotaurin und/oder deren unbedenkliche Salze in Kombination mit mindestens einer Fettsäure und/oder mit einem Extrakt, der solche aufweist, verwendet werden.

7. Verwendung nach Anspruch 6, **dadurch gekennzeichnet, dass** die Fettsäuren aus den essentiellen mehrfach ungesättigten n-6- und n-3-Fettsäuren, welche zwischen 18 und 22 Kohlenstoffatome aufweisen, aus sowie deren Estern und deren Mischungen ausgewählt sind.

8. Verwendung nach einem der vorhergehenden Ansprüche, **dadurch gekennzeichnet, dass** die Zusammensetzung in Form eines Nahrungsergänzungsmittels vorliegt.

9. Verwendung nach einem der Ansprüche 6 bis 8, **dadurch gekennzeichnet, dass** die Polyphenole aus den Procyanidinen, Proanthocyanidinen und deren Mischungen ausgewählt sind.

10. Verwendung nach einem der Ansprüche 6 bis 9, **dadurch gekennzeichnet, dass** die Tagesdosis an Polyphenol(en) im Bereich von 0,5 bis 2.000 mg/Tag liegt.

11. Verwendung nach einem der Ansprüche 6 bis 10, **dadurch gekennzeichnet, dass** die Tagesdosis an Polyphenolen im Bereich von 0,5 bis 1.000 mg/Tag liegt.

12. Verwendung nach einem der Ansprüche 6 bis 11, **dadurch gekennzeichnet, dass** die Fettsäure(n) aus Linolsäure, γ-Linolensäure, Linolensäure, Stearidonsäure, Crocetin und 5,8,11,14-Eicosatetraensäure und deren Mischungen ausgewählt sind.

13. Verwendung nach einem der Ansprüche 6 bis 12, **dadurch gekennzeichnet, dass** die Tagesdosis an Fettsäuren im Bereich von 0,5 bis 3.500 mg/Tag, vorzugsweise von 5 bis 1.500 mg/Tag, liegt.

14. Verwendung nach einem der Ansprüche 6 bis 13, **dadurch gekennzeichnet, dass** die Tagesdosis an n-6-Fettsäuren im Bereich von 0,5 bis 2.600 mg/Tag, vorzugsweise von 5 bis 1.200 mg/Tag, liegt.

15. Verwendung nach einem der Ansprüche 6 bis 14, **dadurch gekennzeichnet, dass** die Tagesdosis an n-3-Fettsäuren im Bereich von 0,5 bis 2.500 mg/Tag, vorzugsweise von 5 bis 360 mg/Tag, liegt.

16. Verwendung nach einem der vorhergehenden Ansprüche, **dadurch gekennzeichnet, dass** die orale Zusammensetzung darüber hinaus mindestens eines der Ergänzungsmittel aufweist, welche aus den Vitaminen C und E, Zink und dessen Salzen, Selen und mindestens einem Carotinoid ausgewählt sind, vorzugsweise aus einem Carotinoid, das aus β-Carotin, Lycopin, Zeaxanthin und Lutein ausgewählt ist.

17. Zusammensetzung zur oralen Aufnahme, **dadurch gekennzeichnet, dass** sie mindestens ein Polyphenol, das aus einem Procyanidin, einem Proanthocyanidin, einer Mischung von Catechinmonomeren mit Oligomeren von Procyanidinen (OPC) sowie aus deren Mischungen und/oder aus einem Extrakt, der solche aufweist, ausgewählt ist; in Kombination mit Taurin und/oder Hypotaurin und/oder deren unbedenklichem Salzen umfasst.

18. Zusammensetzung nach dem vorhergehenden Anspruch, **dadurch gekennzeichnet, dass** sie als Wirkstoff, welcher zur oralen Aufnahme bestimmt ist, mindestens 0,05 bis 80 Gewichts-% an Taurin und/oder an Hypotaurin und/oder an deren unbedenklichen Salzen, in Kombination mit mindestens einem Polyphenol oder mit einem Extrakt, der solche aufweist, sowie einen Hilfsstoff aufweist, wobei die Zusammensetzung frei von Vitamin C ist.

19. Zusammensetzung nach Anspruch 17, **dadurch gekennzeichnet, dass** sie mindestens eine Fettsäure und/oder eines von deren unbedenklichen Salzen aufweist.

20. Zusammensetzung nach dem vorhergehenden Anspruch, **dadurch gekennzeichnet, dass** sie 0,01 bis 10 Gewichts-% mindestens eines Polyphenols und/oder 0,1 bis 10 Gewichts-% mindestens einer Fettsäure umfasst.

21. Zusammensetzung nach Anspruch 17, **dadurch gekennzeichnet, dass** sie mindestens Polyphenole in Kombination mit Taurin und/oder Hypotaurin und/oder deren unbedenklichen Salzen in einem Polyphenol/Taurin-Gewichtsverhältnis von mindestens 0,5, insbesondere von mindestens 0,75, ganz besonders von mindestens 1 umfasst.

22. Zusammensetzung nach Anspruch 17, **dadurch gekennzeichnet, dass** sie 0,01 bis 30 Gewichts-% an Taurin und/oder an Hypotaurin und/oder an deren unbedenklichen Salzen in Kombination mit 0,1 bis 50 Gewichts-% an Extrakten umfasst, die mindestens ein Polyphenol aufweisen.

23. Zusammensetzung nach Anspruch 17, **dadurch gekennzeichnet, dass** sie mindestens Catechine in Kombination mit Taurin und/oder Hypotaurin und/oder deren unbedenklichen Salzen umfasst.

24. Zusammensetzung nach Anspruch 23, **dadurch gekennzeichnet, dass** sie 0,01 bis 30 Gewichts-% an Taurin und/oder an Hypotaurin und/oder an deren unbedenklichen Salzen in Kombination mit 0,1 bis 25 Gewichts-% an Catechinen umfasst.

25. Zusammensetzung nach Anspruch 17, **dadurch gekennzeichnet, dass** sie darüber hinaus mindestens eine der Verbindungen aufweist, welche aus den Vitaminen C und E, Zink oder dessen Salzen, Selen und mindestens einem Carotinoid ausgewählt sind, vorzugsweise aus einem Carotinoid, das aus β-Carotin, Lycopin, Zeaxanthin und Lutein ausgewählt ist.

26. Zusammensetzung nach Anspruch 17, **dadurch gekennzeichnet, dass** der Hilfsstoff für ein Nahrungsergänzungsmittel unbedenklich ist, welches als Dragee, Gelkapsel, Gel, Emulsion, Tablette, Kapsel, Trinkampulle, Pulver zur Verdünnung oder zum Direktverzehr, Nahrungsriegel oder in Form angereicherter Lebensmittel vorliegt.

27. Zusammensetzung nach Anspruch 17, **dadurch gekennzeichnet, dass** es sich um ein Nahrungsergänzungsmittel handelt.

28. Kombination von Taurin und/oder Hypotaurin und/oder unbedenklichen Salzen mit mindestens einem Polyphenol und/oder einem Extrakt, der solche aufweist, wobei sie dazu bestimmt ist, derart verwendet zu werden, dass sie im Rahmen der Behandlung und Verhütung von Haarausfall oral verabreicht wird;
wobei das Polyphenol aus einem Procyanidin, einem Proanthocyanidin, einer Mischung von Catechinmonomeren mit Oligomeren von Procyanidinen (OPC) sowie aus deren Mischungen ausgewählt ist.

29. Kombination, die für eine Verwendung nach Anspruch 28 bestimmt ist; wobei Taurin, Hypotaurin oder deren unbedenkliche Salze in einer Dosis von 0,5 bis 4.000 mg pro Tag als Taurin-Äquivalent verabreicht werden können.

30. Kombination, die für eine Verwendung nach Anspruch 28 oder 29 bestimmt ist; wobei Taurin, Hypotaurin und/oder deren unbedenkliche Salze in Kombination mit mindestens einem der Ergänzungsmittel verwendet werden, welche aus Zink, Fettsäuren, Vitamin C, Vitamin E, einem (oder mehreren) Carotinoid(en), beispielsweise β-Carotin oder Lycopin, ausgewählt sind.

31. Kosmetische Verwendung von Taurin und/oder Hypotaurin und/oder deren unbedenklichen Salzen, in Kombination mit mindestens einem Polyphenol und/oder einem Extrakt, der solche aufweist, zur Verabreichung auf oralem Wege, um das Haar zu behandeln;
wobei das Polyphenol aus einem Procyanidin, einem Proanthocyanidin, einer Mischung von Catechinmonomeren mit Oligomeren von Procyanidinen (OPC) sowie aus deren Mischungen ausgewählt ist.

32. Kosmetische Verwendung von Taurin und/oder Hypotaurin und/oder deren unbedenklichen Salzen, in Kombination mit mindestens einem Polyphenol und/oder mit einem Extrakt, der solche aufweist, zur Verabreichung auf oralem Wege, um die Alterung des Haars zu behandeln oder dieser vorzubeugen;
wobei das Polyphenol aus einem Procyanidin, einem Proanthocyanidin, einer Mischung von Catechinmonomeren mit Oligomeren von Procyanidinen (OPC) sowie aus deren Mischungen ausgewählt ist.

## Claims

1. Cosmetic use of taurine and/or hypotaurine and/or salts thereof that are acceptable, in combination with at least one polyphenol and/or an extract containing the same, for oral administration, for maintaining a good head of hair by acting on the hair density and by reducing the heterogeneity of the hair diameters;
said polyphenol being chosen from a procyanidin, a proanthocyanidin, a mixture of catechin monomers with procyanidin oligomers (PCO), and also mixtures thereof.

2. Use according to Claim 1, **characterized in that** the daily dose of taurine and/or hypotaurine and/or acceptable salts thereof, as taurine equivalent, is between 0.5 and 4000 mg/day.

3. Use according to either of the preceding claims, **characterized in that** the daily dose of taurine and/or hypotaurine and/or acceptable salts thereof, as taurine equivalent, is between 10 and 500 mg/day.

4. Use according to one of the preceding claims, **characterized in that** the daily dose of taurine and/or hypotaurine and/or acceptable salts thereof, as taurine equivalent, is from 50 to 150 mg/day.

5. Use according to one of the preceding claims, **characterized in that** the acceptable salts of taurine and/or hypotaurine are the magnesium, manganese, iron II or zinc salts.

6. Use according to one of the preceding claims, **characterized in that** the taurine and/or hypotaurine and/or acceptable salts thereof are used in combination with at least one fatty acid, and/or an extract comprising the same.

7. Use according to Claim 6, **characterized in that** the fatty acids are chosen from n-6 and n-3 essential polyunsaturated fatty acids, containing between 18 and 22 carbon atoms, and also esters thereof, and mixtures thereof.

8. Use according to one of the preceding claims, **characterized in that** the composition is in the form of a food supplement.

9. Use according to one of Claims 6 to 8, **characterized in that** the polyphenols are chosen from procyanidins, proanthocyanidins, and mixtures thereof.

10. Use according to one of Claims 6 to 9, **characterized in that** the daily dose of polyphenol(s) is between 0.5 and 2000 mg/day.

11. Use according to one of Claims 6 to 10, **characterized in that** the daily dose of polyphenols is from about 0.5 to 1000 mg/day.

12. Use according to one of Claims 6 to 11, **characterized in that** the fatty acid(s) is (are) chosen from linoleic acid, γ-linolenic acid, linolenic acid, stearidonic acid, crocetin and 5,8,11,14-eicosatetraenoic acid, and mixtures thereof.

13. Use according to one of Claims 6 to 12, **characterized in that** the daily dose of fatty acids is between 0.5 and 3500 mg/day and preferably between 5 and 1500 mg/day.

14. Use according to one of Claims 6 to 13, **characterized in that** the dose of n-6 fatty acids is between 0.5 and 2600 mg/day and preferably 5 to 1200 mg/day.

15. Use according to one of Claims 6 to 14, **characterized in that** the dose of n-3 fatty acids is between 0.5 and 2500 mg/day and preferably 5 to 360 mg/day.

16. Use according to one of the preceding claims, **characterized in that** the oral composition also comprises at least one of the supplements chosen from vitamins C and E, zinc or its salts, selenium and at least one carotenoid, preferably a carotenoid chosen from β-carotene, lycopene, zeaxanthin and lutein.

17. Composition for oral absorption, **characterized in that** it comprises at least one polyphenol chosen from a procyanidin, a proanthocyanidin, a mixture of catechin monomers with procyanidin oligomers (PCO), and also mixtures thereof, and/or an extract comprising the same; in combination with taurine and/or hypotaurine and/or acceptable salts thereof.

18. Composition according to the preceding claim, **characterized in that** it comprises, as active agent, at least 0.05% to 80% by weight of taurine and/or hypotaurine and/or acceptable salts thereof, in combination with at least one polyphenol or an extract containing the same, for oral absorption and an excipient, said composition being free of vitamin C.

19. Composition according to Claim 17, **characterized in that** it comprises at least one fatty acid and/or an acceptable salt thereof.

20. Composition according to the preceding claim, **characterized in that** it comprises from 0.01% to 10% by weight of at least one polyphenol and/or 0.1% to 10% by weight of at least one fatty acid.

21. Composition according to Claim 17, **characterized in that** it comprises at least some polyphenols in combination with taurine and/or hypotaurine and/or acceptable salts thereof in a polyphenol/taurine weight ratio at least equal to 0.5, in particular greater than or equal to 0.75 and especially greater than or equal to 1.

22. Composition according to Claim 17, **characterized in that** it comprises 0.01% to 30% by weight of taurine and/or hypotaurine and/or acceptable salts thereof, in combination with 0.1% to 50% by weight of extracts comprising at least one polyphenol.

23. Composition according to Claim 17, **characterized in that** it comprises at least some catechins in combination with taurine and/or hypotaurine and/or acceptable salts thereof.

24. Composition according to Claim 23, **characterized in that** it comprises 0.01% to 30% by weight of taurine and/or hypotaurine and/or acceptable salts thereof, in combination with 0.1% to 25% by weight of catechins.

25. Composition according to Claim 17, **characterized in that** it also comprises at least one of the compounds chosen from vitamins C and E, zinc or its salts, selenium and at least one carotenoid, preferably a carotenoid chosen from β-carotene, lycopene, zeaxanthin and lutein.

26. Composition according to Claim 17, **characterized in that** the excipient is acceptable for a food supplement, in the form of a sugarcoated tablet, a gel capsule, a gel, an emulsion, a tablet, a wafer capsule, a drinkable ampule, a dilutable or nondilutable powder, dietary bars or enriched foods.

27. Composition according to Claim 17, **characterized in that** it is a food supplement.

28. Combination of taurine and/or hypotaurine and/or acceptable salts, with at least one polyphenol and/or an extract containing the same, intended for use, for oral administration, in the treatment and prevention of alopecia;
said polyphenol being chosen from a procyanidin, a proanthocyanidin, a mixture of catechin monomers with procyanidin oligomers (PCO), and also mixtures thereof.

29. Combination intended for use according to Claim 28; in which the taurine, hypotaurine or acceptable salts thereof are administrable at a dose of from 0.5 to 4000 mg per day, as taurine equivalent.

30. Combination intended for use according to Claim 28 or 29; in which the taurine, hypotaurine and/or acceptable salts thereof are used in combination with at least one of the supplements chosen from zinc, fatty acids, vitamin C, vitamin E, and one (or more) carotenoid(s), for example β-carotene or lycopene.

31. Cosmetic use of taurine and/or hypotaurine and/or salts thereof that are acceptable, in combination with at least one polyphenol and/or an extract containing the same, for oral administration, for treating the scalp;
said polyphenol being chosen from a procyanidin, a proanthocyanidin, a mixture of catechin monomers with procyanidin oligomers (PCO), and also mixtures thereof.

32. Cosmetic use of taurine and/or hypotaurine and/or salts thereof that are acceptable, in combination with at least one polyphenol and/or an extract containing the same, for oral administration, for treating and preventing the aging of the hair; said polyphenol being chosen from a procyanidin, a proanthocyanidin, a mixture of catechin monomers with procyanidin oligomers (PCO), and also mixtures thereof.
